(19) **Europäisches Patentamt / European Patent Office / Office européen des brevets**

(11) **EP 4 700 128 A1**

(12) **EUROPEAN PATENT APPLICATION**
published in accordance with Art. 153(4) EPC

(43) Date of publication:
**25.02.2026 Bulletin 2026/09**

(21) Application number: **24797472.8**

(22) Date of filing: **26.04.2024**

(51) International Patent Classification (IPC):
*C12P 7/6409* (2022.01)   *C12N 1/16* (2026.01)
*A23D 9/007* (2006.01)   *A23K 20/158* (2016.01)
*A23K 10/16* (2016.01)   *A61K 8/9728* (2017.01)
*A23L 33/14* (2016.01)   *A61K 36/06* (2006.01)

(52) Cooperative Patent Classification (CPC):
A23D 9/007; A23K 10/16; A23K 20/158;
A23L 33/14; A61K 8/9728; A61K 36/06;
C12N 1/16; C12P 7/6409

(86) International application number:
**PCT/KR2024/005687**

(87) International publication number:
**WO 2024/225818 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.04.2023 KR 20230056288**

(71) Applicant: **CJ Cheiljedang Corporation**
**Seoul 04560 (KR)**

(72) Inventors:
• **LEE, In**
  **Seoul 04560 (KR)**
• **LEE, Peter**
  **Seoul 04560 (KR)**
• **HONG, Sung Ho**
  **Seoul 04560 (KR)**
• **HONG, Jin Tae**
  **Seoul 04560 (KR)**
• **AN, Jungap**
  **Seoul 04560 (KR)**

(74) Representative: **Meissner Bolte Partnerschaft mbB**
**Patentanwälte Rechtsanwälte**
**Postfach 86 06 24**
**81633 München (DE)**

Remarks:
The complete document including Reference Table(s) and the Sequence Listing(s) can be downloaded from the EPO website

(54) **MICROBIAL OIL COMPOSITION COMPRISING RETINOIDS AND FATTY ACIDS**

(57)   The present disclosure relates to a microbial oil composition comprising retinoids and fatty acids; a cosmetic composition comprising the microbial oil composition; a food composition comprising the microbial oil composition; a feed composition comprising the microbial oil composition; and a preparation method thereof.

EP 4 700 128 A1

**Description**

**[Technical Field]**

The present disclosure relates to a microbial oil composition comprising retinoids and fatty acids; a cosmetic composition; a food composition; a feed composition comprising the same; and a preparation method thereof.

**[Background Art]**

Retinoids are essential vitamins that can be involved in improving wrinkles, treating skin diseases, strengthening eye health, such as improving night blindness, *etc.,* and enhancing immunity. In general, retinoids are chemically synthesized and sold on the market (US 2007-0232810 A1, *etc.).* However, raw materials used for retinoids are expensive and have large price fluctuations, and the preparation method thereof poses economic and environmental issues due to complex purification steps and byproduct production. In addition, it is difficult to produce products containing high concentrations of retinoids using conventional methods. Recently, research on fermentation-derived retinoids is underway due to negative perceptions about the chemical synthesis of functional substances such as retinoids and consumer demand for economical/stable prices.

**[Disclosure]**

**[Technical Problem]**

The technical problem to be solved in the present disclosure is to provide a microbial oil composition comprising retinoids and fatty acids; a cosmetic composition; a food composition; a feed composition comprising the same; and a preparation method thereof.

**[Technical Solution]**

It is one object of the present disclosure to provide a microbial oil composition comprising retinoids and fatty acids.
It is another object of the present disclosure to provide a cosmetic composition, a food composition, or a feed composition comprising the microbial oil composition.
It is still another object of the present disclosure to provide a method for preparing the microbial oil composition.

**[Advantageous Effects]**

The microbial oil containing a high content of retinoids can be effective in industry.

**[Detailed Description of Preferred Embodiments]**

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Additionally, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.
One aspect of the present disclosure provides a microbial oil composition comprising retinoids and fatty acids, wherein the retinoids are contained in an amount of 30% by weight or more and less than 100% by weight, and the fatty acids are contained in an amount of greater than 0% by weight and 40% by weight or less, relative to the total oil composition.
As used herein, the term "microbial oil" means an oil derived from microorganisms, and may be an oil contained in microorganisms and/or cultures thereof. The culture of the present disclosure may be a medium obtained by culturing a specific microorganism in a culture medium, a culture solution, an extract, a diluent, a concentrate, a filtrate, a dry product, a lysate obtained by collecting and disrupting cells of the microorganism, a fermented product, a purified product, and/or any type of formulation that may be formed using the same. The culture medium means that it contains a specific microorganism, and the culture filtrate means that it substantially does not contain the specific microorganism (here, it means that the specific microorganism to be separated by filtration, *etc.* is substantially excluded, but does not mean that the microorganism is completely excluded from the filtrate.). Additionally, the microbial oil may be obtained by extracting microorganisms and/or cultures thereof with a non-polar solvent, for example, but not limited to, hexane, ethyl acetate, chloroform, dichloromethane, acetonitrile, butanol, or a combination thereof.

Conventional methods of chemically producing retinoids have the disadvantage of difficulty in producing high concentrations of retinoids due to complicated purification steps and by-product production. Additionally, even if retinoids are produced using a microbial culture, it is known that it is difficult to produce microbial oils containing high concentrations of retinoids because small amounts of retinoids are produced by microorganisms.

As used herein, "retinoids" are a class of chemical compounds of vitamin A or are chemically related to it.

The retinoid may be any one or more selected from the group consisting of retinol, retinal, retinoic acid, and retinyl ester, and the retinyl ester may be any one or more selected from the group consisting of retinyl palmitate and retinyl acetate, but is not limited thereto.

In one embodiment, the microbial oil composition of the present disclosure may contain retinoids in an amount of 30% by weight or more and less than 100% by weight, 30 to 99.9% by weight, 30 to 99.5% by weight, 30 to 99% by weight, 30 to 98% by weight, 30 to 95% by weight, 30 to 90% by weight, 30 to 85% by weight, 30 to 80% by weight, 30 to 75% by weight, 30 to 70% by weight, 30 to 65% by weight, 30 to 60% by weight, 30 to 55% by weight, 30 to 50% by weight, 30 to 49.5% by weight, 30 to 49.3% by weight, 30 to 49% by weight, 30 to 48% by weight, 30 to 47.1% by weight, 30 to 47% by weight, 30 to 46% by weight, 30 to 45.5% by weight, 30 to 45.2% by weight, 30 to 45% by weight, 30 to 44.5% by weight, 30 to 44.3% by weight, 30 to 44% by weight, 40% by weight or more and less than 100% by weight, 40 to 99.9% by weight, 40 to 99.5% by weight, 40 to 99% by weight, 40 to 98% by weight, 40 to 95% by weight, 40 to 90% by weight, 40 to 85% by weight, 40 to 80% by weight, 40 to 75% by weight, 40 to 70% by weight, 40 to 65% by weight, 40 to 60% by weight, 40 to 55% by weight, 40 to 50% by weight, 40 to 49.5% by weight, 40 to 49.3% by weight, 40 to 49% by weight, 40 to 48% by weight, 40 to 47.1% by weight, 40 to 47% by weight, 40 to 46% by weight, 40 to 45.5% by weight, 40 to 45.2% by weight, 40 to 45% by weight, 40 to 44.5% by weight, 40 to 44.3% by weight, 40 to 44% by weight, 44% by weight or more and less than 100% by weight, 44 to 99.9% by weight, 44 to 99.5% by weight, 44 to 99% by weight, 44 to 98% by weight, 44 to 95% by weight, 44 to 90% by weight, 44 to 85% by weight, 44 to 80% by weight, 44 to 75% by weight, 44 to 70% by weight, 44 to 65% by weight, 44 to 60% by weight, 44 to 55% by weight, 44 to 50% by weight, 44 to 49.5% by weight, 44 to 49.3% by weight, 44 to 49% by weight, 44 to 48% by weight, 44 to 47.1% by weight, 44 to 47% by weight, 44 to 46% by weight, 44 to 45.5% by weight, 44 to 45.2% by weight, 44 to 50% by weight, or 44.3 to 49.3% by weight, relative to the total microbial oil composition, but is not limited thereto.

In one embodiment of the above-described embodiments, the retinoids may be contained in an amount of 95% by weight or more and less than 100% by weight, 95 to 99.9% by weight, 95 to 99.8% by weight, 95 to 99.5% by weight, 95 to 99% by weight, 95 to 98% by weight, 96% by weight or more and less than 100% by weight, 96 to 99.9% by weight, 96 to 99.8% by weight, 96 to 99.5% by weight, 96 to 99% by weight, 96 to 98% by weight, 97% by weight or more and less than 100% by weight, 97 to 99.9% by weight, 97 to 99.8% by weight, 97 to 99.5% by weight, 97 to 99% by weight, 97 to 98% by weight, 98% by weight or more and less than 100% by weight, 98 to 99.9% by weight, 98 to 99.8% by weight, 98 to 99.5% by weight, 98 to 99% by weight, or 99% by weight or more and less than 100% by weight, relative to the total retinoids, but is not limited thereto.

In one embodiment of the above-described embodiments, the retinol may be trans-retinol, and may contain 99 to 100% by weight of trans-retinol relative to the total retinol.

In one embodiment of the above-described embodiments, the retinoids may contain greater than 0% by weight and 1% by weight or less, greater than 0% by weight and 0.9% by weight or less, greater than 0% by weight and 0.8% by weight or less, greater than 0% by weight and 0.5% by weight or less, greater than 0% by weight and 0.1% by weight or less, greater than 0% by weight and 0.01% by weight or less, 0.01 to 1 % by weight, 0.01 to 0.9 % by weight, 0.01 to 0.5 % by weight, 0.01 to 0.1 % by weight, 0.1 to 1 % by weight, 0.1 to 0.9 % by weight, 0.1 to 0.8 % by weight, or 0.1 to 0.5 % by weight of each of retinal, retinoic acid, retinyl palmitate, and retinyl acetate, relative to the total retinoids, but is not limited thereto.

In one embodiment, the fatty acid of the present disclosure may be C12 or more and C22 or less (that is, a carbon number of 12 to 22.)

In one embodiment of the above-described embodiments, the fatty acid may be any one or more selected from the group consisting of lauric acid (C12:0), myristic acid (C14:0), pentadecenoic acid (C15:1), palmitic acid (C16:0), hexadecenoic acid (C16:1), heptadecanoic acid (C17:0), heptadecenoic acid (C17:1), stearic acid (C18:0), elaidic acid (C18:1T9), oleic acid (C18:1C9), linoleic acid (C18:2C), arachidic acid (C20:0), heneicosylic acid (C21:0), and docosadienoic acid (C22:2), but is not limited thereto.

In one embodiment, the microbial oil composition of the present disclosure may contain fatty acids in an amount of greater than 0% by weight and 40% by weight or less, greater than 0% by weight and 30% by weight or less, greater than 0% by weight and 25% by weight or less, greater than 0% by weight and 23% by weight or less, greater than 0% by weight and 22% by weight or less, greater than 0% by weight and 21.7% by weight or less, greater than 0% by weight and 21.64% by weight or less, greater than 0% by weight and 21.6% by weight or less, greater than 0% by weight and 21% by weight or less, greater than 0% by weight and 20% by weight or less, greater than 0% by weight and 19% by weight or less, greater than 0% by weight and 18% by weight or less, greater than 0% by weight and 17% by weight or less, greater than 0% by weight and 16% by weight or less, greater than 0% by weight and 15.5% by weight or less, greater than 0% by weight and 15.3% by weight or less, greater than 0% by weight and 15.29% by weight or less, greater than 0% by weight and 15.2% by weight or less, greater than 0% by weight and 15% by weight or less, greater than 0% by weight and 10% by weight or less, greater than 0% by weight and 9% by weight or less, greater than 0% by weight and 8.5% by weight or less, greater than 0% by weight and 8.3% by weight or less, greater than 0% by weight and 8% by weight or less, greater than 0% by weight and 7% by weight or less, greater than 0% by weight and 6.9% by weight or less, greater than 0% by weight and 6.86% by weight or less, greater than 0% by weight and 6.8% by weight or less, greater than 0% by weight and 6.5% by weight or less, greater than 0% by weight and 5% by weight or less, greater than 0% by weight and 3% by weight or less, greater than 0% by weight and 1% by weight or less, 0.1 to 40% by weight, 0.1 to 30% by weight, 0.1 to 25% by weight, 0.1 to 23% by weight, 0.1 to 22% by weight, 0.1 to 21.7% by weight, 0.1 to 21.64% by weight, 0.1 to 21.6% by weight, 0.1 to 21% by weight, 0.1 to 20% by weight, 0.1 to 19% by weight, 0.1 to 18% by weight, 0.1 to 17% by weight, 0.1 to 16% by weight, 0.1 to 15.5% by weight, 0.1 to 15.3% by weight, 0.1 to 15.29% by weight, 0.1 to 15.2% by weight, 0.1 to 15% by weight, 0.1 to 10% by weight, 0.1 to 9% by weight, 0.1 to 8.5% by weight, 0.1 to 8.3% by weight, 0.1 to 8% by weight, 0.1 to 7% by weight, 0.1 to 6.9% by weight, 0.1 to 6.86% by weight, 0.1 to 6.8% by weight, 0.1 to 6.5% by weight, 0.1 to 5% by weight, 0.1 to 3% by weight, 0.1 to 1% by weight, 5 to 40% by weight, 5 to 30% by weight, 5 to 25% by weight, 5 to 23% by weight, 5 to 22% by weight, 5 to 21.7% by weight, 5 to 21.64% by weight, 5 to 21.6% by weight, 5 to 21% by weight, 5 to 20% by weight, 5 to 19% by weight, 5 to 18% by weight, 5 to 17% by weight, 5 to 16% by weight, 5 to 15.5% by weight, 5 to 15.3% by weight, 5 to 15.29% by weight, 5 to 15.2% by weight, 5 to 15% by weight, 5 to 10% by weight, 5 to 9% by weight, 5 to 8.5% by weight, 5 to 8.3% by weight, 5 to 8% by weight, 5 to 7% by weight, 5 to 6.9% by weight, 5 to 6.86% by weight, 5 to 6.8% by weight, 6 to 22% by weight, 6.8 to 21.7% by weight, 6.86 to 21.64% by weight, 8 to 22% by weight, 8.3 to 21.7% by weight, 8.3 to 21.64% by weight, 15 to 22% by weight, 15.2 to 21.7% by weight, 15.29 to 21.64% by weight, 6 to 16% by weight, 6.8 to 15.3% by weight, 6.86 to 15.29% by weight, 8 to 16% by weight, 8.3 to 15.3% by weight, 8.3 to 15.29% by weight, 6 to 9% by weight, 6.8 to 8.3% by weight, 6.86 to 8.3% by weight, relative to the total microbial oil composition, but is not limited thereto.

In one embodiment, the microbial oil composition of the present disclosure may contain 20% by weight or more of oleic acid, 30% by weight or less of linoleic acid, and 30% by weight or less of palmitic acid, relative to the total fatty acids, but is not limited thereto.

Specifically, the microbial oil composition of the present disclosure may contain 20 to 70% by weight of oleic acid, 5 to 30% by weight of linoleic acid, and 5 to 30% by weight of palmitic acid, relative to the total fatty acids, but is not limited thereto.

In one embodiment of the above-described embodiments, the microbial oil composition of the present disclosure may contain 20 to 70% by weight, 20 to 60% by weight, 20 to 50% by weight, 20 to 40% by weight, 20 to 30% by weight, 22 to 67% by weight, 22 to 66% by weight, 22 to 48% by weight, 22 to 47% by weight, 22 to 27% by weight, 22 to 26% by weight, 26 to 66% by weight, 26 to 48% by weight, 26 to 47% by weight, 30 to 70% by weight, 30 to 60% by weight, 30 to 50% by weight, 30 to 40% by weight, 40 to 70% by weight, 40 to 60% by weight, 40 to 50% by weight, 40 to 45% by weight, 47 to 67% by weight, 47 to 66% by weight, 50 to 70% by weight, 50 to 60% by weight, or 60 to 70% by weight of oleic acid, relative to the total fatty acids, but is not limited thereto.

In one embodiment of the above-described embodiments, the microbial oil composition of the present disclosure may contain 5 to 30% by weight, 5 to 25% by weight, 5 to 20% by weight, 5 to 15% by weight, 5 to 10% by weight, 10 to 30% by weight, 10 to 25% by weight, 10 to 21% by weight, 10 to 20% by weight, 10 to 19% by weight, 10 to 15% by weight, 10 to 13% by weight, 10 to 11% by weight, 15 to 30% by weight, 15 to 25% by weight, 15 to 20% by weight, 19 to 23% by weight, 19 to 21% by weight, 20 to 30% by weight, or 20 to 25% by weight of linoleic acid and/or palmitic acid, relative to the total fatty acids, but is not limited thereto.

The microorganism of the microbial oil of the present disclosure may be a microorganism having a retinoid-producing ability.

As used herein, the term "microorganism" or "strain" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.*, and may be a microorganism including genetic modification to produce a desired polypeptide, protein, or product.

The microorganism of the present disclosure may be a microorganism having a retinoid-producing ability. The 'microorganism having a retinoid-producing ability' may be used interchangeably with 'microorganism producing retinoid'. The microorganism having a retinoid-producing ability may be a microorganism naturally having a retinoid-producing ability or a microorganism in which genetic modification has occurred, thus having a retinoid-producing ability.

The microorganism of the present disclosure may be a microorganism of the genus *Yarrowia,* and specifically, may be *Yarrowia lipolytica,* but is not limited thereto.

The microorganism having a retinoid-producing ability may be a microorganism in which a polynucleotide encoding lycopene cyclase/phytoene synthase (crtYB) and phytoene desaturase (crtI) has been introduced into the microorganism to have a retinoid-producing ability in a microorganism not endogenously having a retinoid-producing ability or to further enhance the retinoid-producing ability in a microorganism having a retinoid-producing ability, thereby exhibiting the activity of these proteins or enhancing the activity of these proteins. The lycopene cyclase/phytoene synthase or phytoene desaturase may be a protein derived from *Xanthophyllomyces dendrorhous,* but any protein having the same or similar activity may be included without limitation. In one embodiment, lycopene cyclase/phytoene synthase or phytoene desaturase may consist of or comprise an amino acid sequence of SEQ ID NO: 1 or SEQ ID NO: 3, respectively, but may also consist of or comprise an amino acid sequence, which has a homology or identity of at least 60%, 65%, 70%, 75%, 80%, 85%, 86%, 87%, 88%, 89%, 90%, 91%, 92%, 93%, 94%, 95%, 96%, 97%, 98%, or 99% with the above amino acid sequence and exhibits activity corresponding to the lycopene cyclase/phytoene synthase or phytoene desaturase. Additionally, it is apparent that any protein having an amino acid sequence, in which part of the sequence is deleted, modified, substituted, or added, may also fall within the scope of the lycopene cyclase/phytoene synthase or phytoene desaturase as long as the amino acid sequence has such homology or identity, and exhibits activity corresponding to the lycopene cyclase/phytoene synthase or phytoene desaturase. Further, in one embodiment, the polynucleotide encoding the lycopene cyclase/phytoene synthase or phytoene desaturase may consist of or comprise a sequence of SEQ ID NO: 2 or SEQ ID NO: 4, respectively.

The polynucleotide of the present disclosure may undergo various modifications in the coding region within the scope that does not change the amino acid sequence, due to codon degeneracy or in consideration of the codons preferred in an organism. Specifically, the polynucleotide may consist of or comprise a nucleotide sequence having a homology or identity of 60% or more, 70% or more, 80% or more, 85% or more, 90% or more, 95% or more, 96% or more, 97% or more, 98% or more, and less than 100% to the sequence of SEQ ID NO: 2 or SEQ ID NO: 4, but is not limited thereto.

The microorganism of the present disclosure may be a microorganism in which a polynucleotide encoding geranylgeranyl pyrophosphate synthase (GGPPS) has been introduced into the microorganism to have a retinoid-producing ability in a microorganism not endogenously having a retinoid-producing ability or to further enhance the retinoid-producing ability in a microorganism having a retinoid-producing ability, thereby exhibiting the activity of geranylgeranyl pyrophosphate synthase or enhancing the activity of geranylgeranyl pyrophosphate synthase. The geranylgeranyl pyrophosphate synthase may be a protein derived from *Haematococcus pluvialis,* but any protein having the same or similar activity may be included without limitation. In one embodiment, the geranylgeranyl pyrophosphate synthase may consist of or comprise an amino acid sequence of SEQ ID NO: 5, but may also consist of or comprise an amino acid sequence, which has a homology or identity of at least 60%, 65%, 70%, 75%, 80%, 85%, 86%, 87%, 88%, 89%, 90%, 91%, 92%, 93%, 94%, 95%, 96%, 97%, 98%, or 99% with the above amino acid sequence and exhibits activity corresponding to the geranylgeranyl pyrophosphate synthase. Additionally, it is apparent that any protein having an amino acid sequence, in which part of the sequence is deleted, modified, substituted, or added, may also fall within the scope of the geranylgeranyl pyrophosphate synthase as long as the amino acid sequence has such homology or identity, and exhibits activity corresponding to the geranylgeranyl pyrophosphate synthase. Further, in one embodiment, the polynucleotide encoding the geranylgeranyl pyrophosphate synthase may have or comprise a sequence of SEQ ID NO: 6. The polynucleotide of the present disclosure may undergo various modifications in the coding region within the scope that does not change the amino acid sequence, due to codon degeneracy or in consideration of the codons preferred in an organism. Specifically, the polynucleotide may consist of or comprise a nucleotide sequence having a homology or identity of 60% or more, 70% or more, 80% or more, 85% or more, 90% or more, 95% or more, 96% or more, 97% or more, 98% or more, and less than 100% to the sequence of SEQ ID NO: 6, but is not limited thereto.

Additionally, the microorganism of the present disclosure may be a microorganism in which a polynucleotide encoding beta-carotene 15,15'-oxygenase (BLH) has been introduced into the microorganism to have a retinoid-producing ability in a microorganism not endogenously having a retinoid-producing ability or to further enhance the retinoid-producing ability in a microorganism having a retinoid-producing ability, thereby exhibiting the activity of beta-carotene 15,15'-oxygenase or enhancing the activity of beta-carotene 15,15'-oxygenase. The beta-carotene 15,15'-oxygenase may be a protein derived from an uncultured marine bacterium 66A03, but any protein having the same or similar activity may be included without limitation. In one embodiment, the beta-carotene 15,15'-oxygenase may consist of or comprise an amino acid sequence of SEQ ID NO: 7, but may also consist of or comprise an amino acid sequence, which has a homology or identity of at least 60%, 65%, 70%, 75%, 80%, 85%, 86%, 87%, 88%, 89%, 90%, 91%, 92%, 93%, 94%, 95%, 96%, 97%, 98%, or 99% with the above amino acid sequence and exhibits activity corresponding to the beta-carotene 15,15'-oxygenase. Additionally, it is apparent that any protein having an amino acid sequence, in which part of the sequence is deleted, modified, substituted, or added, may also fall within the scope of the beta-carotene 15,15'-oxygenase as long as the amino acid sequence has such homology or identity, and exhibits activity corresponding to the beta-carotene 15,15'-oxygenase. Further, in one embodiment, the polynucleotide encoding the beta-carotene 15,15'-oxygenase may have or comprise a sequence of SEQ ID NO: 8. The polynucleotide of the present disclosure may undergo various modifications in the coding region within the scope that does not change the amino acid sequence, due to codon degeneracy or in consideration of the codons preferred in an organism. Specifically, the polynucleotide may consist of or comprise a nucleotide sequence having a homology or identity of 60% or more, 70% or more, 80% or more, 85% or more, 90% or more, 95% or more, 96% or more, 97% or more, 98% or more, and less than 100% to the sequence of SEQ ID NO: 8, but is not limited thereto.

In one embodiment, CC08-2050(KCCM13294P), one of the microorganisms having a retinoid-producing ability of the present disclosure, may comprise the lycopene cyclase/phytoene synthase comprising the amino acid sequence of SEQ ID NO: 1; the phytoene desaturase comprising the amino acid sequence of SEQ ID NO: 3; the geranylgeranyl pyrophosphate synthase comprising the amino acid sequence of SEQ ID NO: 5; and the beta-carotene 15,15'-oxygenase comprising the amino acid sequence of SEQ ID NO: 7.

The microorganism having a retinoid-producing ability of the present disclosure may be CC08-2050(KCCM13294P, Ref. Park et al., Metabolic engineering 2022;73:26-37), but is not limited thereto. The CJ2050 strain described in the above reference (Park et al., Metabolic engineering 2022;73:26-37) has been deposited under the name of CC08-2050 strain of the present disclosure, and the CC08-2050 strain of the present disclosure and the CJ2050 are identical strains.

The microbial oil composition of the present disclosure may further comprise any suitable excipient commonly used in the art, and such excipients may comprise, for example, preservatives, wetting agents, dispersing agents, suspending agents, buffers, stabilizers, or isotonic agents, *etc.*, but are not limited thereto.

Another aspect of the present disclosure provides a cosmetic composition comprising the microbial oil composition of the present disclosure.

The cosmetic composition may be used for all purposes (*e.g.,* whitening, wrinkle improvement, *etc.*) for which retinoids are used.

The microbial oil composition of the present disclosure may be contained in an amount of 0.001 to 90% based on the total weight of the cosmetic composition.

The cosmetic composition of the present disclosure may be prepared in a form selected from the group consisting of solution, ointment for external use, cream, foam, nutritive cosmetic water, softening cosmetic water, perfume, facial mask, softening water, emulsion, makeup base, essence, soap, liquid washing agent, bath foam, sunscreen cream, sun oil, suspension, emulsion, paste, gel, lotion, powder, soap, surfactant-containing cleanser, oil, powder foundation, emulsion foundation, wax foundation, patch and spray, but is not limited thereto.

The cosmetic composition of the present disclosure may further comprise one or more cosmetically acceptable carriers mixed with general skin cosmetics. As common ingredients, for example, buffers, oil, water, surfactants, moisturizers, lower alcohols, thickening agents, chelating agents, colorings, preservatives, fragrances, *etc.* may be appropriately mixed, but are not limited thereto.

Still another aspect of the present disclosure provides a food composition comprising the microbial oil composition of the present disclosure.

The food composition may be used for all purposes for which retinoids are used. The microbial oil composition of the present disclosure may be contained in an amount of 0.001 to 90% based on the total weight of the food composition. The food composition may comprise buffers, food-acceptable carriers, excipients, stabilizers and/or additives, *etc.* known in the art.

Still another aspect of the present disclosure provides a feed composition comprising the microbial oil composition of the present disclosure.

The feed composition may be used for all purposes for which retinoids are used. The microbial oil composition of the present disclosure may be contained in an amount of 0.001 to 90% based on the total weight of the feed composition. The feed composition may comprise buffers, feed-acceptable carriers, excipients, stabilizers and/or additives, *etc.* known in the art.

Still another aspect of the present disclosure provides a pharmaceutical composition comprising the microbial oil composition of the present disclosure.

The pharmaceutical composition may be used for all purposes for which retinoids are used. The microbial oil composition of the present disclosure may be contained in an amount of 0.001 to 90% based on the total weight of the pharmaceutical composition. The pharmaceutical composition may comprise buffers, pharmaceutically acceptable carriers, excipients, stabilizers and/or additives, *etc.* known in the art

In the present disclosure, the type of carrier is not particularly limited, and any carrier may be used as long as it is commonly used in the relevant technical field and is acceptable in the cosmetic, food, feed, and/or pharmaceutical industries.

Still another aspect of the present disclosure provides a method for preparing a microbial oil composition comprising retinoids and fatty acids, comprising culturing a microorganism having a retinoid-producing ability in a medium.

The microorganism, retinoids, fatty acids, and microbial oil composition are as described in other aspects.

As used herein, the term "cultivation" means that the microorganism of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the microorganism to be selected. Specifically, the cultivation may be a batch culture, a continuous culture, and/or a fed-batch culture, but is not limited thereto.

The microorganism of the present disclosure may be cultured under aerobic conditions in a conventional medium containing an appropriate carbon source, nitrogen source, phosphorus source, inorganic compound, amino acid, and/or vitamin, *etc.,* while adjusting temperature, pH, *etc.*

In the present disclosure, the carbon source may comprise carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.*; and amino acids, such as glutamic acid, methionine, lysine, *etc.* Additionally, the carbon source may comprise natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.,* molasses converted to reducing sugar) may be used, and in addition, various other carbon sources in an appropriate amount may be used without limitation. These carbon sources may be used alone or in a combination of two or more kinds, but are not limited thereto.

The nitrogen source may comprise inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may comprise monopotassium phosphate, dipotassium phosphate, or corresponding sodium-containing salts, *etc.* Examples of the inorganic compounds may comprise sodium chloride, calcium chloride, iron chloride, magnesium sulfate, iron sulfate, manganese sulfate, calcium carbonate, *etc.* Additionally, amino acids, vitamins, and/or appropriate precursors, *etc.* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected into the medium in order to maintain aerobic conditions of the medium; or nitrogen gas, hydrogen gas, or carbon dioxide may be injected to maintain anaerobic or microaerobic conditions, without the injection of gas, but the gas is not limited thereto.

Additionally, the culture medium may contain metal salts, such as magnesium sulfate or iron sulfate required for growth. Lastly, in addition to the above substances, essential growth materials such as amino acids and vitamins may be used. Additionally, appropriate precursors may be added to the culture medium. These materials may be added to the culture in the form of a batch culture or continuous culture during the cultivation process, but are not limited thereto.

In the present disclosure, the pH of the culture may be adjusted by adding a compound such as ammonium hydroxide, potassium hydroxide, ammonia, phosphoric acid, sulfuric acid, *etc.* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected into the medium in order to maintain aerobic conditions of the medium; or nitrogen gas, hydrogen gas, or carbon dioxide may be injected to maintain anaerobic or microaerobic conditions, without the injection of gas, but the method is not limited thereto.

The temperature of the culture of the present disclosure may be maintained in the range from 20°C to 35°C and specifically from 25°C to 35°C. The cultivation may be continued until the production of a useful material can be obtained, may be carried out for about 10 to 160 hours, about 20 to 130 hours, about 24 to 120 hours, about 36 to 120 hours, about 48 to 120 hours, about 48 hours or more, or about 48 hours, about 72 hours, or about 120 hours, but is not limited thereto.

The method for preparing a microbial oil composition of the present disclosure may further comprise a step of recovering microbial oil from the microorganism, medium, or culture.

In the recovering step, the desired microbial oil may be collected using the method of culturing the microorganism of the present disclosure, for example, using a suitable method known in the relevant technical field according to a batch culture, continuous culture, or fed-batch culture method, *etc.* For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, but are not limited thereto.

In one embodiment, the recovering step may comprise a step of extracting microbial oil from the microorganism or the culture.

The extraction method of the present disclosure is not particularly limited, and extraction can be performed according to a method commonly used in the relevant technical field. The type of solvent used for the extraction is not particularly limited, and any solvent known in the relevant technical field may be used. Non-limiting examples of the extraction solvent may include, but are not limited to, hexane, ethyl acetate, chloroform, dichloromethane, acetonitrile, butanol, or a combination thereof.

The method may comprise an additional purification process. The purification process may be performed by a suitable method known in the relevant technical field.

**[Mode for Carrying Out the Invention]**

Hereinafter, the present disclosure will be described in detail by way of Examples. However, these Examples are merely preferred Examples given for illustrative purposes, and thus, the scope of the invention is not intended to be limited to or by these Examples. Meanwhile, technical features that are not described herein can be sufficiently understood and easily carried out by those skilled in the art in the technical field of the present disclosure or in a similar technical field.

**Example 1. Preparation of Strains for Preparing Microbial Oil**

CC08-2050 (CJ2050, *Yarrowia lipolytica,* Park et al., Metabolic engineering 2022;73:26-37), which is a strain capable of producing retinoids belonging to the genus *Yarrowia,* deposited with Accession No. KCCM13294P, was used for the preparation of microbial oils.

**Example 2. Preparation of Microbial Oil 1**

**2.1. Preparation of Fermented Liquid**

The *Yarrowia lipolytica* strain of Example 1 was cultured in a 5 L fermenter for 120 hours. Prior to culture in the fermenter, the strain was cultured at 30°C and 200 rpm for 48 hours in a 250 ml flask containing a YLMM2 (*Yarrowia lipolytica* minimal media 2) medium sterilized for the purpose of a seed culture. The flask culture containing the seed culture was dispensed and inoculated into a 5 L fermenter, and cultured in a sterilized YLMM2 medium under conditions of 30°C, 900 rpm, 1.0 vvm, and pH 6.5.

<YLMM2 (pH7.2)>

The YLMM2 medium was prepared by dissolving 40 g of glucose, 1.7 g of yeast nitrogen base without amino acids and ammonium sulfate, 0.5 g of uracil and 1.25 g of ammonium sulfate in 1L of 0.1 M sodium phosphate buffer (pH 7.2).

**2.2. Extraction of Microbial Oils**

Solvent extraction of microbial oil was performed using n-hexane, which is the most commonly used solvent for extraction. The solvent extraction was performed by adding the same volume of solvent as the fermented liquid prepared in Example 2.1 to a 1,000 ml sealed container, and then performing the extraction in a shaking water bath (Jeiotech) at 30°C and 150 rpm for 3 hours.

1 L of the above extract was added to an evaporator (EYELA, N-1210B), and the solvent was recovered under conditions of 30°C and 100 mmHg to obtain microbial oil (Microbial Oil 1) from which the solvent was removed. The solvent removal process was performed at 25 to 35°C with maximum light blocking to prevent the destruction of retinoids.

**Example 3. Preparation of Microbial Oil 2**

**3.1. Preparation of Fermented Liquid**

The *Yarrowia lipolytica* strain of Example 1 was cultured in a 5 L fermenter for 120 hours. The strain was cultured at 30°C and 200 rpm for 48 hours in a 250 ml flask containing a YLMM2 (*Yarrowia lipolytica* minimal media 2) medium sterilized for the purpose of a seed culture. The flask culture containing the seed culture was dispensed and inoculated into a 5 L fermenter, and cultured in a sterilized YLMM2 medium supplemented with 2% by weight of Tween 20 under conditions of 30°C, 900 rpm, 1.0 vvm, and pH 6.5.

### 3.2. Extraction of Microbial Oil

A microbial oil was obtained from the fermented liquid of Example 3.1 in the same manner as in Example 2.2.

## Example 4. Preparation of Microbial Oils 3 and 4

### 4.1. Preparation of Fermented Liquid

The *Yarrowia lipolytica* strain of Example 1 was cultured in a 5 L fermenter for 120 hours. Prior to culture in the fermenter, the strain was cultured at 30°C and 250 rpm for 48 hours in a 250 ml flask containing a YLMM2 (*Yarrowia lipolytica* minimal media 2) medium sterilized for the purpose of a seed culture. The flask culture containing the seed culture was dispensed and inoculated into a 5 L fermenter, and cultured in a sterilized YLMM2 medium supplemented with 2% by weight of Tween 20 under conditions of 30°C, 900 rpm, 1.0 vvm, and pH 6.5.

### 4.2. Extraction of Microbial Oil

Prior to extraction, the microbial cells were separated from the fermented liquid. The microbial cells were separated using a centrifuge at 4,000 rpm for 10 minutes, and microbial oils were extracted from the separated microbial cells and the permeate in the same manner as in Example 2.2 to obtain microbial oil 3 (microbial cells) and oil 4 (permeate). The microbial oil from the microbial cells was extracted by adding the same volume of extraction solvent as the fermented liquid according to Example 4.1 before the separation of microbial cells.

## Example 5. Analysis of Crude Fat Content and Fatty Acid Profile of Microbial Oils

The Roese-Gottlieb method was used to measure the fat content of microbial oils 1 to 4 prepared in Examples 2 to 4.

To this end, 5 g of each sample of microbial oils 1 to 4 was placed in a Mojonnier tube, and water was added to adjust the volume to about 11 ml, heated to 45°C, and thoroughly mixed with shaking. 1.5 ml of ammonia water was added thereto and mixed, and then 10 ml of alcohol was added thereto and mixed. Then, 25 ml of ether was added thereto, mixed, and vortexed for 1 minute. Thereafter, the resultant was centrifuged at 600 rpm, the supernatant was transferred to a pre-weighed Erlenmeyer flask, 15 ml of ether was added to the remaining liquid in the tube, and extraction was performed three more times in the same manner as above. Afterwards, the outlet of the tube plug and the funnel were washed thoroughly with ether, and the filtrate and the washed liquid were mixed in an Erlenmeyer flask to evaporate the solvent in a water bath, and then the resultant was dried in a 100° C dryer until the weight became constant, and the crude fat was extracted. The crude fat content was calculated using the following calculation formula.

- Crude Fat Content (%) = Weight of Extracted Crude Fat (g) / Weight of Microbial Oil (g) x 100

To confirm the fatty acid composition of the extracted crude fat liquid, the fatty acid methyl esterification method (FAME) was used. 15 to 20 mg of dried crude fat was placed in a 15 ml vial, and 2 ml of ISTD (standard material, prepared by dissolving 0.1 g of glyceryl triundecanoate in 100 ml of 2,2,4-trimethylpentane) and 1.5 ml of 0.5 N NaOH dissolved in methanol were added. The vial was immediately capped and vortexed. Afterwards, the vial was reacted in a constant-temperature water bath at 85°C for 30 minutes and cooled to room temperature. After adding 5 ml of saturated sodium chloride solution, 2 ml of 2,2,4-trimethylpentane was added, and vortexed. The supernatant was added to a 1.5 ml tube containing a small amount of anhydrous sodium sulfate ($Na_2SO_4$) prepared in advance, mixed, and then only the supernatant was subjected to GC-FID analysis. The analyzed fatty acids are shown in Table 1 below in % content in the sample (C16:0 palmitic acid, C18:1C9 oleic acid, C18:2C linoleic acid).

[Table 1]

|  | Microbia l Oil 1 | Microbia l Oil 2 | Microbia l Oil 3 | Microbia l Oil 4 | Microbial Oil 1 | Microbia l Oil 2 | Microbia l Oil 3 | Microbial Oil 4 |
|---|---|---|---|---|---|---|---|---|
|  | wt % in Microbial Oils | | | | wt % in TFA | | | |
| Crude Oil | 99.5 | 99.0 | 99.3 | 99.9 | | | | |
| TFA | 8.30 | 15.29 | 21.64 | 6.86 | 100.00 | 100.00 | 100.00 | 100.00 |

(continued)

|  | Microbia l Oil 1 | Microbia l Oil 2 | Microbia l Oil 3 | Microbia l Oil 4 | Microbial Oil 1 | Microbia l Oil 2 | Microbia l Oil 3 | Microbial Oil 4 |
|---|---|---|---|---|---|---|---|---|
|  | wt % in Microbial Oils | | | | wt % in TFA | | | |
| Crude Oil | 99.5 | 99.0 | 99.3 | 99.9 |  |  |  |  |
| C4:0 | 0.00 | 0.00 | 0.00 | 0.00 | 0.00 | 0.00 | 0.00 | 0.00 |
| C6:0 | 0.00 | 0.00 | 0.00 | 0.00 | 0.00 | 0.00 | 0.00 | 0.00 |
| C8:0 | 0.00 | 0.00 | 0.00 | 0.00 | 0.00 | 0.00 | 0.00 | 0.00 |
| C10:0 | 0.00 | 0.00 | 0.00 | 0.00 | 0.00 | 0.00 | 0.00 | 0.00 |
| C11:0 | 0.00 | 0.00 | 0.00 | 0.00 | 0.00 | 0.00 | 0.00 | 0.00 |
| C12:0 | 0.06 | 0.00 | 3.55 | 0.21 | 0.72 | 0.00 | 16.40 | 3.06 |
| C13:0 | 0.00 | 0.00 | 0.00 | 0.00 | 0.00 | 0.00 | 0.00 | 0.00 |
| C14:0 | 0.10 | 0.00 | 1.79 | 0.04 | 1.20 | 0.00 | 8.27 | 0.58 |
| C14:1 | 0.00 | 0.00 | 0.00 | 0.00 | 0.00 | 0.00 | 0.00 | 0.00 |
| C15:0 | 0.00 | 0.00 | 0.00 | 0.00 | 0.00 | 0.00 | 0.00 | 0.00 |
| C15:1 | 0.09 | 0.00 | 0.00 | 0.18 | 1.08 | 0.00 | 0.00 | 2.62 |
| C16:0 | 1.58 | 1.59 | 4.62 | 1.60 | 19.04 | 10.39 | 21.35 | 23.32 |
| C16:1 | 0.25 | 0.88 | 2.05 | 0.62 | 3.01 | 5.77 | 9.47 | 9.04 |
| C17:0 | 0.05 | 0.00 | 0.00 | 0.00 | 0.60 | 0.00 | 0.00 | 0.00 |
| C17:1 | 0.05 | 0.00 | 0.00 | 0.04 | 0.60 | 0.00 | 0.00 | 0.58 |
| C18:0 | 0.93 | 0.25 | 2.31 | 0.85 | 11.20 | 1.66 | 10.67 | 12.39 |
| C18:1T9 | 0.14 | 0.00 | 0.00 | 0.06 | 1.69 | 0.00 | 0.00 | 0.87 |
| C18:1C9 | 3.94 | 10.12 | 4.82 | 1.83 | 47.47 | 66.17 | 22.27 | 26.68 |
| C18:2T | 0.00 | 0.00 | 0.00 | 0.00 | 0.00 | 0.00 | 0.00 | 0.00 |
| C18:2C | 0.85 | 2.06 | 2.49 | 1.35 | 10.24 | 13.48 | 11.51 | 19.68 |
| C20:0 | 0.00 | 0.00 | 0.00 | 0.04 | 0.00 | 0.00 | 0.00 | 0.58 |
| C18:3n6 | 0.00 | 0.00 | 0.00 | 0.00 | 0.00 | 0.00 | 0.00 | 0.00 |
| C20:1 | 0.00 | 0.00 | 0.00 | 0.00 | 0.00 | 0.00 | 0.00 | 0.00 |
| C18:3n3 | 0.00 | 0.00 | 0.00 | 0.00 | 0.00 | 0.00 | 0.00 | 0.00 |
| C21:0 | 0.12 | 0.37 | 0.00 | 0.00 | 1.45 | 2.45 | 0.00 | 0.00 |
| C20:2 | 0.00 | 0.00 | 0.00 | 0.00 | 0.00 | 0.00 | 0.00 | 0.00 |
| C22:0 | 0.00 | 0.00 | 0.00 | 0.00 | 0.00 | 0.00 | 0.00 | 0.00 |
| C20:3n6 | 0.00 | 0.00 | 0.00 | 0.00 | 0.00 | 0.00 | 0.00 | 0.00 |
| C22:1 | 0.00 | 0.00 | 0.00 | 0.00 | 0.00 | 0.00 | 0.00 | 0.00 |
| C20:3n3 | 0.00 | 0.00 | 0.00 | 0.00 | 0.00 | 0.00 | 0.00 | 0.00 |
| C23:0 | 0.00 | 0.00 | 0.00 | 0.00 | 0.00 | 0.00 | 0.00 | 0.00 |
| C20:4 | 0.00 | 0.00 | 0.00 | 0.00 | 0.00 | 0.00 | 0.00 | 0.00 |
| C22:2 | 0.04 | 0.00 | 0.00 | 0.04 | 0.48 | 0.00 | 0.00 | 0.58 |
| C24:0 | 0.00 | 0.00 | 0.00 | 0.00 | 0.00 | 0.00 | 0.00 | 0.00 |
| C20:5n3 | 0.00 | 0.00 | 0.00 | 0.00 | 0.00 | 0.00 | 0.00 | 0.00 |
| C24:1 | 0.00 | 0.00 | 0.00 | 0.00 | 0.00 | 0.00 | 0.00 | 0.00 |

(continued)

|  | Microbia l Oil 1 | Microbia l Oil 2 | Microbia l Oil 3 | Microbia l Oil 4 | Microbial Oil 1 | Microbia l Oil 2 | Microbia l Oil 3 | Microbial Oil 4 |
|---|---|---|---|---|---|---|---|---|
|  | wt % in Microbial Oils | | | | wt % in TFA | | | |
| Crude Oil | 99.5 | 99.0 | 99.3 | 99.9 | | | | |
| C22:6n3 | 0.00 | 0.00 | 0.00 | 0.00 | 0.00 | 0.00 | 0.00 | 0.00 |

## Example 6. Analysis of Retinoid Content in Microbial Oils

In order to measure the retinoid content in microbial oils 1 to 4 prepared in Examples 2 to 4, 0.5 ml of acetone (Sigma, Cas Number 67-64-1) containing 4% BHT (Sigma, Cas Number 128-37-0) was added to each sample of microbial oils 1 to 4, and the mixture was shaken (2,000 rpm) at 45 °C for 15 minutes. The extracted retinoids were quantitatively analyzed using HPLC equipment, and the results are shown in Table 2 below.

[Table 2]

|  | Microbial Oil 1 | Microbial Oil 2 | Microbial Oil 3 | Microbial Oil 4 |
|---|---|---|---|---|
|  | wt % in Microbial Oils | | | |
| Retinoids | 47.1 | 44.3 | 45.2 | 49.3 |
| - Retinol | 47.1 | 44.3 | 45.2 | 49.3 |
| - Retinal | 0.0 | 0.0 | 0.0 | 0.0 |
| - Retinyl Palmitate | 0.0 | 0.0 | 0.0 | 0.0 |
| - Retinyl Acetate | 0.0 | 0.0 | 0.0 | 0.0 |
| - Retinoic Acid | 0.0 | 0.0 | 0.0 | 0.0 |
| Retinols | wt % in Retinols | | | |
| - Trans-retinol | 100 | 100 | 100 | 100 |
| - Cis-retinol | 0.0 | 0.0 | 0.0 | 0.0 |

From the above results, it was confirmed in the present disclosure that microbial oils containing a high content of retinoids can be prepared.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the present disclosure is defined by the appended claims rather than by the description preceding them, and therefore all changes and modifications that fall within the metes and bounds of the claims, or equivalents of such metes and bounds, are therefore intended to be embraced by the claims.

**EP 4 700 128 A1**

EP 24797472.8
Our Ref.: M/CJG-154-PC/EP

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
　　CJ CHEILJEDANG CENTER,
　　330, DONGHO-RO,
　　JUNG-GU, SEOUL 100-400,
　　REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

| I . IDENTIFICATION OF THE MICROORGANISM |
|---|

| Identification reference given by the DEPOSITOR: <br><br> *Yarrowia lipolytica* CC08-2050 | Accession number given by the INTERNATIONAL DEPOSITARY AUTHORITY: <br><br> KCCM13294P |
|---|---|

| II . SCIENTIFIC DESCRIPTION AND/OR PROPOSED TAXONOMIC DESIGNATION |
|---|
| The microorganism identified under I above was accompanied by: <br> ☐ a scientific description <br> ☐ a proposed taxonomic designation <br> (Mark with a cross where applicable) |

| III. RECEIPT AND ACCEPTANCE |
|---|
| This International Depositary Authority accepts the microorganism identified under I above, which was received by it on December. 01. 2022　(date of the original deposit).[1] |

| IV. RECEIPT OF REQUEST FOR CONVERSION |
|---|
| The microorganism identified under I above was received by this International Depositary Authority on　　　　　(date of the original deposit) and a request to convert the original deposit to a deposit under the Budapest Treaty was received by it on　　　　　(date of receipt of request for conversion). |

| V . INTERNATIONAL DEPOSITARY AUTHORITY |
|---|

| Name : Korean Culture Center of Microorganisms <br><br> Address : Yurim B/D <br>　　　　45, Hongjenae-2ga-gil <br>　　　　Seodaemun-gu <br>　　　　SEOUL 03641 <br>　　　　Republic of Korea | Signature(s) of person(s) having the power to represent the International Depositary Authority or of authorized official(s): <br><br> Date: December. 01. 2022. |
|---|---|

[1] Where Rule 6.4(d) applies, such date is the date on which the status of international depositary authority was acquired.

Form BP/4 (sole page)

한국미생물보존센터 사단법인 한국종균협회
03641 서울특별시 서대문구 홍제내2가길 45 유림빌딩　Tel: 02-391-0950, 396-0950　Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea.　Tel: 82-2-391-0950, 396-0950　Fax: 82-2-392-2859

**Claims**

1. A microbial oil composition comprising retinoids and fatty acids, wherein the retinoids are contained in an amount of 30% by weight or more and less than 100% by weight, and the fatty acids are contained in an amount of greater than 0% by weight and 40% by weight or less, relative to the total oil composition.

2. The microbial oil composition of claim 1, wherein the fatty acid is C12 or more and C22 or less.

3. The microbial oil composition of claim 1, wherein the fatty acid is any one or more selected from the group consisting of lauric acid (C12:0), myristic acid (C14:0), pentadecenoic acid (C15:1), palmitic acid (C16:0), hexadecenoic acid (C16:1), heptadecanoic acid (C17:0), heptadecenoic acid (C17:1), stearic acid (C18:0), elaidic acid (C18:1T9), oleic acid (C18:1C9), linoleic acid (C18:2C), arachidic acid (C20:0), heneicosylic acid (C21:0), and docosadienoic acid (C22:2).

4. The microbial oil composition of claim 1, wherein the fatty acids comprise 20 to 70% by weight of oleic acid, 5 to 30% by weight of linoleic acid, and 5 to 30% by weight of palmitic acid, relative to the total fatty acids.

5. The microbial oil composition of claim 1, wherein the retinoids comprise 95% by weight or more and less than 100% by weight of retinol, greater than 0% by weight and 1% by weight or less of retinal, greater than 0% by weight and 1% by weight or less of retinoic acid, greater than 0% by weight and 1% by weight or less of retinyl palmitate, and greater than 0% by weight and 1% by weight or less of retinyl acetate, relative to the total retinoids.

6. The microbial oil composition of claim 5, wherein the retinol comprises 99 to 100% by weight of trans-retinol relative to the total retinols.

7. The microbial oil composition of claim 1, wherein the microorganism has a retinoid-producing ability.

8. The microbial oil composition of claim 1, wherein the microorganism is a microorganism of the genus *Yarrowia*.

9. The microbial oil composition of claim 1, wherein the microorganism is *Yarrowia lipolytica*.

10. A cosmetic composition comprising the microbial oil composition of claim 1.

11. A food composition comprising the microbial oil composition of claim 1.

12. A feed composition comprising the microbial oil composition of claim 1.

13. A pharmaceutical composition comprising the microbial oil composition of claim 1.

14. A method for preparing a microbial oil composition comprising retinoids and fatty acids, comprising culturing a microorganism having a retinoid-producing ability in a medium.

# EP 4 700 128 A1

## INTERNATIONAL SEARCH REPORT

| | International application No. |
|---|---|
| | **PCT/KR2024/005687** |

---

**A.    CLASSIFICATION OF SUBJECT MATTER**

**C12P 7/6409**(2022.01)i; **C12N 1/16**(2006.01)i; **A23D 9/007**(2006.01)i; **A23K 20/158**(2016.01)i; **A23K 10/16**(2016.01)i; **A61K 8/9728**(2017.01)i; **A23L 33/14**(2016.01)i; **A61K 36/06**(2006.01)i

According to International Patent Classification (IPC) or to both national classification and IPC

---

**B.    FIELDS SEARCHED**

Minimum documentation searched (classification system followed by classification symbols)

C12P 7/6409(2022.01); A61K 8/37(2006.01); C12N 1/20(2006.01); C12P 1/04(2006.01); C12P 7/64(2006.01); C12P 7/6463(2022.01); C12P 7/6472(2022.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 레티노이드(retinoid), 레티놀(retinol), 비타민 A(vitamin A), 지방산(fatty acid), 미생물(microorganism), 오일(oil)

---

**C.    DOCUMENTS CONSIDERED TO BE RELEVANT**

| Category* | Citation of document, with indication, where appropriate, of the relevant passages | Relevant to claim No. |
|---|---|---|
| A | WO 2022-104063 A2 (C16 BIOSCIENCES, INC.) 19 May 2022 (2022-05-19)<br>See claims 1, 63, 73 and 79. | 1-14 |
| A | KR 10-2013-0014445 A (INDUSTRY-ACADEMIC COOPERATION FOUNDATION GYEONGSANG NATIONAL UNIVERSITY) 07 February 2013 (2013-02-07)<br>See claim 1. | 1-14 |
| A | KR 10-2022-0143872 A (C16 BIOSCIENCES, INC.) 25 October 2022 (2022-10-25)<br>See claims 1, 6 and 9-11. | 1-14 |
| A | KR 10-2016-0102008 A (DSM IP ASSETS B.V.) 26 August 2016 (2016-08-26)<br>See paragraph [0021]; and claims 1 and 16. | 1-14 |
| A | PARK, H. et al. Efficient production of retinol in Yarrowia lipolytica by increasing stability using antioxidant and detergent extraction. Metabolic engineering. 2022, vol. 73, pp. 26-37 (published online: 06 June 2022).<br>See abstract. | 1-14 |

---

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

---

| Date of the actual completion of the international search | Date of mailing of the international search report |
|---|---|
| **05 August 2024** | **07 August 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/005687**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

International application No.

**PCT/KR2024/005687**

| Patent document cited in search report | | | Publication date (day/month/year) | Patent family member(s) | | | Publication date (day/month/year) |
|---|---|---|---|---|---|---|---|
| WO | 2022-104063 | A2 | 19 May 2022 | EP | 4244372 | A2 | 20 September 2023 |
| | | | | KR | 10-2023-0129160 | A | 06 September 2023 |
| | | | | US | 2024-000697 | A1 | 04 January 2024 |
| | | | | WO | 2022-104063 | A3 | 04 August 2022 |
| KR | 10-2013-0014445 | A | 07 February 2013 | CN | 103857786 | A | 11 June 2014 |
| | | | | CN | 103857786 | B | 06 April 2016 |
| | | | | KR | 10-1359484 | B1 | 13 February 2014 |
| | | | | KR | 10-1392159 | B1 | 12 May 2014 |
| | | | | KR | 10-1440922 | B1 | 18 September 2014 |
| | | | | KR | 10-2013-0014673 | A | 08 February 2013 |
| | | | | KR | 10-2013-0014674 | A | 08 February 2013 |
| | | | | US | 2014-0170720 | A1 | 19 June 2014 |
| | | | | US | 2016-0362709 | A1 | 15 December 2016 |
| | | | | US | 9644217 | B2 | 09 May 2017 |
| | | | | US | 9834794 | B2 | 05 December 2017 |
| | | | | WO | 2013-019051 | A2 | 07 February 2013 |
| | | | | WO | 2013-019051 | A3 | 20 June 2013 |
| | | | | WO | 2013-019052 | A2 | 07 February 2013 |
| | | | | WO | 2013-019052 | A3 | 04 July 2013 |
| | | | | WO | 2013-019053 | A2 | 07 February 2013 |
| | | | | WO | 2013-019053 | A3 | 04 July 2013 |
| KR | 10-2022-0143872 | A | 25 October 2022 | AU | 2021-220796 | A1 | 19 August 2021 |
| | | | | CA | 3167348 | A1 | 19 August 2021 |
| | | | | CN | 115968405 | A | 14 April 2023 |
| | | | | EP | 4103728 | A1 | 21 December 2022 |
| | | | | JP | 2023-513271 | A | 30 March 2023 |
| | | | | US | 2023-203549 | A1 | 29 June 2023 |
| | | | | WO | 2021-163194 | A1 | 19 August 2021 |
| KR | 10-2016-0102008 | A | 26 August 2016 | AU | 2014-369042 | A1 | 25 June 2015 |
| | | | | AU | 2014-369042 | B2 | 30 April 2020 |
| | | | | CN | 105939710 | A | 14 September 2016 |
| | | | | CN | 105939710 | B | 08 May 2020 |
| | | | | EP | 3082792 | A2 | 26 October 2016 |
| | | | | JP | 2017-501709 | A | 19 January 2017 |
| | | | | JP | 2020-054349 | A | 09 April 2020 |
| | | | | JP | 6989094 | B2 | 05 January 2022 |
| | | | | KR | 10-2552228 | B1 | 05 July 2023 |
| | | | | TW | 201529831 | A | 01 August 2015 |
| | | | | US | 10472316 | B2 | 12 November 2019 |
| | | | | US | 2016-0340287 | A1 | 24 November 2016 |
| | | | | WO | 2015-095690 | A2 | 25 June 2015 |
| | | | | WO | 2015-095690 | A3 | 23 December 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070232810 A1

**Non-patent literature cited in the description**

- **PARK et al.** *Metabolic engineering*, 2022, vol. 73, 26-37
- *CHEMICAL ABSTRACTS*, 67-64-1
- *CHEMICAL ABSTRACTS*, 128-37-0